# DEMANDE DE BREVET EUROPEEN

(11) **EP 4 062 907 A1**
(43) Date de publication de la demande: **28.09.2022**
(21) Numéro de dépôt: 21305359.8
(22) Date de dépôt: 23.03.2021
(51) Int. Cl.: A61K 9/16, A61K 47/38, A61P 31/14, A61P 33/14, A61P 33/00

(54) **FORMULATION PAR VOIE ORALE D'IVERMECTINE ET UTILISATIONS**

(71) Demandeur: Substipharm, 75016 Paris (FR)
(72) Inventeur: SUPLIE, Pascal, 27400 MONTAURE (FR); JOUANNY, Eléonore, 92330 SCEAUX (FR)
(74) Mandataire: Regimbeau

(57) **Abrégé**

L'invention a pour objet une formulation galénique, adaptée à une administration par voie orale, comprenant une enveloppe renfermant des mini formulations sous forme solide, ayant une taille inférieure à 4,5 mm, d'ivermectine micronisée; son procédé de préparation et ses utilisations.

## Description

La présente invention concerne une formulation galénique, adaptée à une administration par voie orale, comprenant de l'ivermectine, son procédé de préparation et ses utilisations thérapeutiques.

L'ivermectine appartient à la classe des lactones macrolides ; il s'agit d'un dérivé des avermectines isolé à partir de la fermentation de bouillons de *Streptomyces avermitilis.* Cette molécule présente une affinité importante pour les canaux chlorure glutamate-dépendants présents dans les cellules nerveuses et musculaires des invertébrés.

Sa fixation sur ces canaux favorise une augmentation de la perméabilité membranaire aux ions chlorure entraînant une hyperpolarisation de la cellule nerveuse ou musculaire. Il en résulte une paralysie neuromusculaire pouvant entraîner la mort de certains parasites. L'ivermectine interagit également avec d'autres canaux chlorure ligand-dépendants comme celui faisant intervenir le neuromédiateur GABA (acide gamma-amino-butyrique). Les mammifères ne possèdent pas de canaux chlorure glutamate-dépendants. De plus, les avermectines ne passent pas facilement la barrière hémato méningée ce qui explique leur toxicité relativement faible chez l'Homme.

Ainsi l'ivermectine est efficace contre de nombreux nématodes, acariens et certains insectes également tels que l'anophèle ; c'est un antiparasitaire ancien très largement utilisé en médecine vétérinaire mais aussi historiquement, du fait de sa faible toxicité, chez l'homme en Afrique dans l'onchocercose ou les filarioses. Aujourd'hui elle est également utilisée de façon plus ubiquitaire dans le traitement des pédiculoses et scabiose communément appelée gale.

La gale est une infection due à un sarcopte : le *scabies scabei var. Hominis.* Il s'agit d'une parasitose cutanée contagieuse très répandue à travers le monde avec environ 300 millions de cas par an. Elle touche tous les milieux sociaux économiques cependant elle est souvent associée à la pauvreté, au surpeuplement et à la malnutrition. Elle est pandémique dans les pays tropicaux et en voie de développement. Cette parasitose est très contagieuse et pose un problème important pour les collectivités telles que les EHPAD, écoles, foyers, voire hôpitaux. La gale se manifeste par un prurit à recrudescence nocturne accompagné de lésions spécifiques telles que le sillon scabieux ; Il existe plusieurs formes de sévérité différente, notamment en fonction de l'âge mais dans tous les cas la contagion reste extrêmement problématique.

L'ivermectine est utilisée par voie orale sous forme de comprimés dosés à 3 mg, à raison de 200 µg par kilo. La posologie doit être adaptée en fonction du poids du patient ; un adulte de poids supérieur à 80 kg sera donc amené à prendre 6 comprimés en une fois.

Il existe également des formes topiques (lotions ou crèmes, décrits notamment dans les demandes de brevet EP 1294 375, EP 1620 113, EP 1729 811; EP 2010 148 ; EP 3 019 173 ; EP 3 EP 326 608 ; EP 3 326 609 ; EP 3 326 614 ; EP 2 653 160) et également des préparations type shampoing plus spécifiquement destinées à traiter les pédiculoses. L'ivermectine a été décrite dans le brevet EP 0 001 689. Il s'agit d'un mélange de 22,23-dihydroavermectin B1a (numéro CAS : [71827-03-7]) et de 22,23-dihydroavermectin B1b (numéro CAS : [70209-81-3]), avec
B1a répondant à la formule suivante : 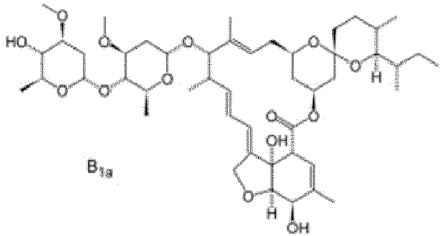 et B1b répondant à la formule suivante :

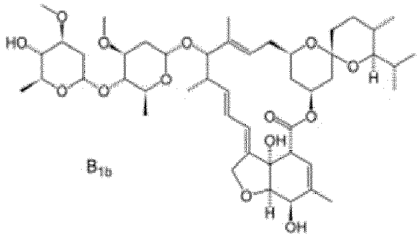

Des comprimés d'ivermectine sont commercialisés sous le nom commercial Stromectol^{®}. Les comprimés de Stromectol^{®} comprennent 3 mg d'ivermectine et les excipients suivants : acide citrique anhydre, amidon de maïs modifié, butylhydroxyanisole, cellulose microcristalline, stéarate de magnésium.

Des comprimés d'ivermectine sont par ailleurs notamment décrits dans les demandes de brevet CN106619685, CN106852930, CN107028892, CN107669646, CN107837238 et CN 108079006.

L'ivermectine donne de bons résultats dans le traitement du paludisme avec des posologies sensiblement équivalentes ou légèrement supérieures et la mise en place d'un traitement de masse en prophylaxie est en cours d'étude.

L'ivermectine et les autres molécules de la classe des lactones macrolides possèdent également une activité antibactérienne et antivirale et récemment de nombreuses études ont été conduites afin de mettre en évidence l'activité antivirale.

Le mécanisme d'action n'est pas encore bien défini mais passerait principalement par une inhibition du transfert du virus dans le cytoplasme des cellules épithéliales via l'ACE2 (enzyme de conversion de l'angiotensine 2) et / ou inhibition du transfert des protéines virales vers le noyau cellulaire. Ainsi, l'ivermectine peut également être utilisée dans des infections bactériennes, telles que les maladies nosocomiales, les infections au staphylocoque doré *(staphylococcus aureus*) et les infections virales telles que la grippe ou les maladies à coronavirus notamment la COVID-19.

Les doses testées sont plus élevées : entre 24 mg et 70 mg minimum par jour et sur plusieurs jours de traitement.

Il apparait donc que l'ivermectine et les molécules apparentées présentent un spectre d'activité très large et en cela très intéressant, d'autant plus que la toxicité associée est assez faible ; cependant les formulations orales existantes imposent une prise répétée et simultanée de plusieurs comprimés ce qui n'est pas propice à une bonne observance du traitement.

Ceci est notamment exacerbé dans le cas de la population âgée ou bien dans les parasitoses aiguës où les patients vont présenter des difficultés à ingérer les prises répétées.

L'autre problématique engendrée est le phénomène de résistance : l'arrêt ou la mauvaise observance du traitement par le patient sont susceptibles de favoriser l'apparition de phénomène de résistance c'est à dire l'émergence de parasites résistants à la molécule, voire à la classe thérapeutique. Il est donc primordial de disposer dans l'arsenal thérapeutique de médicaments efficaces et adaptés.

Par ailleurs, dans le souhait de traiter les infections d'origine bactérienne ou virale, nécessitant des doses plus élevées, une amélioration de la pharmacocinétique est également recherchée.

L'objet de l'invention ici présentée est de répondre à cette problématique et donc de permettre au thérapeute de disposer d'une forme orale multi particulaire et homothétique lui permettant d'ajuster plus facilement la dose au poids du patient et présentant une pharmacocinétique améliorée par rapport au produit de référence, qui est le Stromectol^{®}.

### Résumé de l'invention

L'invention a pour objet une formulation galénique, adaptée à une administration par voie orale, comprenant une enveloppe renfermant des mini formulations sous forme solide, ayant une taille inférieure à 4,5 mm, d'ivermectine micronisée.

Avantageusement, l'ivermectine micronisée a une distribution de tailles de particules dans laquelle le D50 varie entre 1 µm et 30 µm, préférentiellement entre 1 µm et 20 µm. La formulation galénique comprenant une enveloppe est avantageusement choisie parmi des gélules, des sachets ou des sticks.

La formulation galénique selon l'invention comprend avantageusement 3 mg à 75 mg d'ivermectine par formulation, plus avantageusement de plus de 3mg à 75 mg d'ivermectine par formulation, encore plus avantageusement de 12 mg à 50 mg d'ivermectine par formulation, encore plus avantageusement de 12 mg à 24 mg d'ivermectine par formulation.

Avantageusement, la mini formulation solide comprend de 2% à 50% en poids, par rapport au poids total de la mini formulation, d'ivermectine micronisée.

Les mini formulations solides sont avantageusement choisies parmi des microgranules ou des mini comprimés.

Avantageusement, chaque microgranule comprend du centre vers la périphérie
- un support,
- au moins une couche de montage comprenant l'ivermectine micronisée, optionnellement un agent antioxydant, et un agent liant pharmaceutiquement acceptable,
- au moins une couche d'enrobage.

Avantageusement, chaque mini comprimé comprend un diluant, un liant, un agent antioxydant, un lubrifiant, et de l'ivermectine micronisée.

Avantageusement, chaque mini comprimé a une taille inférieure à 3 mm.

L'invention a également pour objet un procédé de préparation d'une formulation galénique selon l'invention, comprenant les étapes suivantes :
a) préparation d'une mini formulation sous forme solide, ayant une taille inférieure à 4,5 mm, d'ivermectine micronisée ;
b) remplissage d'une enveloppe de la formulation avec la quantité désirée de mini formulation solide préparée à l'étape a).

L'invention a également pour objet l'utilisation d'une formulation galénique selon l'invention pour adapter de manière homothétique le dosage en ivermectine en fonction de la posologie.

L'invention a également pour objet l'utilisation d'une formulation galénique selon l'invention dans le traitement ou la prévention de maladies dues ou induites par un parasite, une bactérie, un virus, en particulier :
- dans le traitement ou la prévention des parasitoses, notamment de l'onchocercose, des filarioses, des pédiculoses, ou de la gale ;
- dans le traitement ou la prévention d'infections bactériennes, telles que les maladies nosocomiales, notamment certaines infections au staphylocoque doré *(staphylococcus aureus*) *;*
- dans le traitement ou la prévention d'infections virales telles que la grippe ou les maladies à coronavirus notamment la COVID-19.

### Descriptif de l'invention

L'invention a pour objet une formulation galénique, adaptée à une administration par voie orale, comprenant une enveloppe renfermant des mini formulations sous forme solide, ayant une taille inférieure à 4,5 mm, d'ivermectine micronisée.

Ainsi, la formulation galénique comprend des mini formulations d'ivermectine micronisée. Les mini formulations sont contenues dans une enveloppe. Les mini formulations sont sous forme solide et ont une taille inférieure à 4,5 mm, avantageusement inférieure ou égale à 4 mm, plus avantageusement inférieure ou égale à 3 mm.

Cette formulation permet de proposer plusieurs dosages d'ivermectine et permet notamment d'administrer une dose plus importante d'ivermectine en une seule prise. Cette formulation permet également une adaptation homothétique de la quantité de mini formulations comprises dans l'enveloppe en fonction du dosage recherché. Cette formulation permet également une amélioration de la pharmacocinétique de l'ivermectine, comparée à un comprimé comprenant la même dose en ivermectine

La première étape de fabrication comprend une étape de micronisation de l'ivermectine Par « micronisé », on entend que la taille des particules d'ivermectine est réduite, notamment soit par broyage humide soit par micronisation à sec.

Dans la description de l'invention, sauf indication contraire, l'emploi du terme « ivermectine » fera référence à de l'ivermectine micronisée.

En particulier, l'ivermectine micronisée se caractérise par une distribution de tailles de particules dans laquelle le D50 varie entre 1 µm et 30 µm, préférentiellement entre 1 µm et 20 µm, plus préférentiellement entre 1 µm et 15 µm, encore plus préférentiellement entre 3 µm et 10 µm.

En particulier, l'ivermectine micronisée se caractérise par une distribution de tailles de particules dans laquelle le D10 est inférieur à 10 µm, préférentiellement inférieur à 5 µm, plus préférentiellement inférieur ou égal à 3 µm.

En particulier, l'ivermectine micronisée se caractérise par une distribution de tailles de particules dans laquelle le D90 est inférieur à 100 µm, préférentiellement inférieur à 80 µm, plus préférentiellement inférieur à 50 µm, préférentiellement inférieur à 40 µm, plus préférentiellement inférieur à 30 µm, encore plus préférentiellement inférieur ou égal à 20 µm.

Le D50 est le 50ème centile de la distribution de taille des particules, en volume, c'est-à-dire que 50 % des particules ont une taille inférieure ou égale au D50 et 50 % des particules ont une taille supérieure au D50.

Le D10 est le 10ème centile de la distribution de taille des particules, en volume, c'est-à-dire que 10 % des particules ont une taille inférieure ou égale au D10 et 90 % des particules ont une taille supérieure au D10.

Le D90 est le 90ème centile de la distribution de taille des particules, en volume, c'est-à-dire que 90 % des particules ont une taille inférieure ou égale au D90 et 10 % des particules ont une taille supérieure au D90.

Les distributions de taille de particules et les tailles de particules inférieures sont mesurées par des techniques de diffraction laser, par exemple diffusion de la lumière. Un équipement de diffusion de la lumière est bien connu dans l'art, par exemple un analyseur de taille de particules à diffraction laser Mastersizer 3000.

La réduction de taille peut être obtenue par broyage humide. Les techniques de broyage sont bien connues dans la technique et comprennent le broyage de supports, tel que le broyage de billes, le broyage par vibration ; le broyage à jet, par exemple à l'aide d'un microniseur, d'un broyeur à jet ou d'un pulvérisateur à jet; broyage par dispersion et broyage colloïdal ; l'homogénéisation ; et tout autre technique connue de l'homme du métier.

La formulation galénique comprend une enveloppe permettant de contenir les mini formulations. L'enveloppe peut être toute galénique permettant de renfermer les mini formulations. Cette enveloppe peut être un dispositif permettant de renfermer, avantageusement également de distribuer, les mini formulations, tel que des sachets ou des sticks. L'enveloppe peut également être elle-même adaptée à une administration par voie orale et peut ainsi par exemple être choisie parmi des gélules et les capsules, de préférence les gélules. Les gélules ou capsules ont avantageusement une taille adaptée à une administration par voie orale, et notamment variant du centimètre à quelques centimètres. Les gélules sont le plus souvent des gélules à base de gélatine, des gélules de paraffine, des gélules à base de cellulose (en particulier en hydroxypropylméthylcellullose), des gélules en pullulane, des gélules à base de feuilles d'azyme.

La formulation galénique comprend avantageusement 3 mg à 75 mg d'ivermectine par enveloppe, avantageusement de plus de 3 mg à 75 mg d'ivermectine par enveloppe, plus avantageusement de plus de 3 mg à 50 mg d'ivermectine par enveloppe, encore plus avantageusement de 12 mg à 50 mg d'ivermectine par enveloppe, encore plus avantageusement de 12 mg à 24 mg d'ivermectine par enveloppe.

Ainsi, lorsque par exemple l'enveloppe est une gélule ou une capsule, chaque gélule ou capsule comprend avantageusement 3 mg à 75 mg d'ivermectine par gélule ou capsule, avantageusement de plus de 3 mg à 75 mg d'ivermectine par gélule ou capsule, plus avantageusement de plus de 3 mg à 50 mg d'ivermectine par gélule ou capsule, encore plus avantageusement de 12 mg à 50 mg d'ivermectine par gélule ou capsule, encore plus avantageusement de 12 mg à 24 mg d'ivermectine par gélule ou capsule.

L'ivermectine est formulée sous forme de mini formulations solides qui vont être disposées dans l'enveloppe. Chaque mini formulation constitue une unité galénique.

La mini formulation solide, c'est-à-dire chaque unité galénique, comprend avantageusement 2 à 50% en poids, plus avantageusement 2 à 40% en poids, encore plus avantageusement 2 à 30% en poids, par rapport au poids total de la mini formulation, c'est-à-dire par rapport au poids total d'une unité galénique, d'ivermectine micronisée. La mini formulation solide, c'est-à-dire chaque unité galénique, comprend avantageusement l'ivermectine micronisée comme seul principe actif ayant une activité thérapeutique propre.

La mini formulation solide, c'est-à-dire chaque unité galénique, comprend avantageusement également un agent antioxydant tel que le butylhydroxyanisole (BHA), l'acide citrique, le 2,6-di-tert-butyl-4-méthyl, le propyl gallate, la vitamine E, et leurs combinaisons, avantageusement le BHA.

La teneur en agent antioxydant varie avantageusement de 0,005 à 0,05% en poids, par rapport au poids total de la mini formulation, c'est-à-dire par rapport au poids total d'une unité galénique.

La mini formulation solide, c'est-à-dire chaque unité galénique, peut comprendre un ou plusieurs excipients destinés par exemple à supporter l'ivermectine micronisée, à favoriser le déroulement du processus de préparation tels que des agents anti-collage, des lubrifiants, à améliorer la cohésion tels que les agents liants, des agents de charge, des plastifiants, des agents de remplissage, des tensioactifs, des désintégrants, des édulcorants, des colorants.

Les mini formulations solides sont avantageusement choisies parmi des microgranules ou des mini comprimés.

Les microgranules de la présente invention se rapportent à des unités galéniques sphériques, constituées en leur centre d'un support, recouvert d'au moins une couche contenant le principe actif qui est elle-même recouverte d'au moins une couche polymérique.

Les microgranules de la présente invention peuvent également être obtenus par un procédé en lui-même connu tel que, par exemple, l'extrusion-sphéronisation, la granulation humide ou à chaud.

Ces microgranules peuvent être obtenus de façon classique par dépôt (montage) de l'ivermectine micronisée à la surface de supports.

On entend par "support " ou "noyau", des supports sphériques ou quasi-sphériques de taille comprise entre 50 µm et 800 µm et préférentiellement entre 100 µm et 600 µm tels que ceux habituellement utilisés dans l'industrie pharmaceutique comme support de base de principes actifs pour la constitution de microgranules tels que des microbilles à base de cellulose, obtenues par exemple par extrusion-sphéronisation, ou d'un mélange de sucre et d'amidon et vendues sous le terme "sugar spheres" ou encore des granulés d'autres excipients, comme le lactose par exemple, ou autres supports adaptés à un usage pharmaceutique obtenus par extrusion-sphéronisation. Ces supports sont avantageusement neutres, c'est-à-dire inertes d'un point de vue pharmaceutique. Le support peut également être choisi parmi les composés inertes préalablement décrits recouverts d'une couche polymérique, le polymère pouvant être choisi parmi les polymères à libération prolongée et les polymères désintégrants tels que les gommes, en particulier la gomme xanthane.

Le procédé de dépôt (montage) de l'actif est réalisé de façon classique et connue de l'homme du métier. Ainsi, le dépôt (montage) peut s'effectuer par pulvérisation d'une solution ou suspension d'ivermectine micronisée à la surface du support en alternance avec le dépôt par poudrage d'ivermectine micronisée à la surface du support préalablement humidifié à l'aide d'une solution d'agent liant. Le dépôt (montage) peut également s'effectuer par pulvérisation d'une solution ou suspension comprenant l'ivermectine micronisée et un liant à la surface du support.

Un microgranule selon l'invention comprend avantageusement du centre vers la périphérie
- un support,
- au moins une couche de montage comprenant l'ivermectine micronisée et un agent liant pharmaceutiquement acceptable,
- au moins une couche d'enrobage.

La couche de montage est également appelée couche active.

L'ivermectine micronisée est intégrée dans la couche active en association avec un agent liant pharmaceutiquement acceptable, tel que ceux habituellement utilisés dans l'industrie pharmaceutique pour la fixation de principes actifs à la surface de supports. Parmi les agents liants pharmaceutiquement acceptables, on utilisera préférentiellement des agents liants de nature hydrophile et notamment des dérivés de la cellulose tels que l'HPMC, en particulier les grades Methocel^{®} E5, ou l'hydroxypropylcellulose ou l'hydroxyéthylcellulose, des dérivés de la polyvinylpyrrolidone, et également des dérivés du polyéthylene glycol, des dérivés vinyliques tels que l'alcool polyvinylique.

La teneur en liant pharmaceutique varie avantageusement de 1% à 5% en poids, avantageusement 1 à 3% en poids, par rapport au poids total du microgranule.

De façon préférée, la couche active des microgranules conformes à l'invention est appliquée par pulvérisation d'une dispersion d'ivermectine micronisée dans un solvant (appelée dispersion de montage). Le solvant pourra par exemple être choisi parmi l'eau, des solvants organiques, parmi eux l'éthanol ou des solutions hydro- alcooliques de diverses concentrations. Il est préférable d'utiliser des solvants non toxiques et facilement éliminables par évaporation lors du séchage afin qu'il n'en subsiste aucune trace dans les microgranules.

La couche active peut également comprendre un agent antioxydant tel que décrit précédemment, notamment le butylhydroxyanisole (BHA).

L'enrobage permet de protéger la couche d'ivermectine de l'extérieur, et notamment de l'humidité extérieure. Les microgranules sont de préférence enrobés par un agent d'enrobage choisi dans le groupe constitué de la gomme laque, de la polyvinylpyrrolidone, du polyéthylène glycol (PEG), des dérivés cellulosiques tels que l'hydroxypropylméthylcellulose (HPMC) ou l'hydroxypropylcellulose (HPC), du saccharose, de l'alginate, des glycérides d'acides gras, des polymères méthacryliques, les polymères hydrosolubles. A titre de polymère hydrosoluble on peut en particulier citer l'alcool polyvinylique et ses dérivés.

La couche d'enrobage est avantageusement soluble dans l'eau.

La couche d'enrobage comprend avantageusement également une charge inerte. Avantageusement, la charge inerte uniformément répartie dans la couche d'enrobage est choisie dans le groupe comprenant notamment le talc, le dioxyde de titane, le stéarate de magnésium, le monostéarate de glycérol, la silice et les dérivés des silicates (silicate de magnésium, silicate d'aluminium), le stéarylfumarate de magnésium, les poudres minérales (gypse, zéolithe, pierre, terres diatomées) et leurs mélanges.

Un agent tensioactif est optionnellement présent dans l'enrobage. L'agent tensioactif est de préférence sélectionné dans le groupe de produits suivants : les sels alcalins ou alcalinoterreux des acides gras, le sodium dodécyl sulfate et le docusate de sodium étant préférés, les huiles polyoxyéthylénées, de préférence l'huile de ricin hydrogénée polyoxyéthylénée, les copolymères polyoxyéthylène- polyoxypropylène, les esters de sorbitan polyoxyéthylénés, les dérivés de l'huile de ricin polyoxyéthylénés, les stéarates, de préférence de calcium, de magnésium, d'aluminium ou de zinc, les polysorbates, les stéarylfumarates, de préférence de sodium, le béhénate de glycérol, le chlorure de benzalkonium, le bromure d'acétyltriméthyl ammonium, l'alcool cétylique et leurs mélanges.

Un agent plastifiant est également optionnellement présent dans l'enrobage. L'agent plastifiant est sélectionné notamment dans le groupe de produits suivants : le glycérol et ses esters, de préférence dans le sous-groupe suivant : les triglycérides à chaînes moyennes, les glycérides acétylés, glycéryl-mono- stéarate, glycéryl-thacétate, glycéryl-tributyrate, les phtalates, de préférence dans le sous-groupe suivant : dibutylphtalate, diéthylphtalate, diméthylphtalate, dioctylphtalate, les citrates, de préférence dans le sous-groupe suivant : acétyltributylcitrate, acétylthéthylcitrate, tributylcitrate, triéthylcitrate, les sébacates, de préférence dans le sous-groupe suivant : diéthylsébacate, dibutylsébacate, les adipates, les azélates, les benzoates, le chlorobutanoles polyéthylène glycols, les huiles végétales, les fumarates, de préférence le diéthylfumarate, les malates, de préférence le diéthylmalate, les oxalates, de préférence le diéthyloxalate, les succinates ; de préférence le dibutylsuccinate, les butyrates, les esters de l'alcool cétylique, les malonates, de préférence le diéthylmalonate, l'huile de ricin, et leurs mélanges.

On peut également ajouter dans l'enrobage un ou plusieurs excipients tels que des lubrifiants, des colorants, des édulcorants, des agents anti- adhérants.

La couche d'enrobage comprend de préférence l'HPMC ou un polymère tel que l'alcool polyvinylique. On peut utiliser des produits commerciaux de formulation d'enrobage prêt à l'emploi comprenant un polymère, un plastifiant et un pigment, tels que les produits commerciaux vendus sous la désignation Opadry. Par exemple, le produit commercial Opadry AMB II est une formulation d'enrobage prête à l'emploi comprenant de l'alcool polyvinylique, du talc, du dioxide de titane, du glycerol monocaprylocaprate et du sodium dodécyl sulfate.

Chaque microgranule a avantageusement un diamètre inférieur à 2 mm, avantageusement variant de 0,35 µm à 1 mm.

Avantageusement, les microgranules comprennent, en poids par rapport au poids total de microgranule :
- de l'ivermectine micronisée, dans les quantités décrites précédemment ;
- un support, en une quantité allant de 70% à 90% en poids, par rapport au poids total de microgranule ;
- un liant, en une quantité allant de 1% à 3% en poids, par rapport au poids total de microgranule ;
- un agent antioxydant, en une quantité allant de 0,005% à 0,05% en poids, par rapport au poids total de microgranule ;
- une couche d'enrobage.

Le support est avantageusement constitué de particules de sucre et d'amidon et vendues sous le terme "sugar spheres" ayant un diamètre moyen de 500-600 µm. Il s'agit d'un support neutre. Le liant est avantageusement l'HPMC. L'agent antioxydant est avantageusement le BHA. L'enrobage peut comprendre de l'HPMC ou de l'alcool polyvinylique.

Les microgranules peuvent être préparés par des procédés bien connus de l'homme de l'art. Les microgranules peuvent notamment être préparés par un procédé qui comprend les étapes suivantes :
- l'introduction de supports sphériques s dans une enceinte à lit d'air fluidisé, par exemple,
- la pulvérisation, en lit d'air fluidisé ou en turbine perforée par exemple, sur ces supports sphériques d'ivermectine micronisée en suspension dans un solvant adapté, notamment un solvant hydro-alcoolique, additionné d'au moins un agent liant et optionnellement d'un antioxydant,
- la pulvérisation d'une suspension d'enrobage ou solution d'enrobage, en lit d'air fluidisé ou en turbine perforée par exemple
- éventuellement, le séchage des microgranules ainsi obtenus, par exemple en lit d'air fluidisé ou en turbine perforée puis éventuellement tamisage.

Les mini comprimés de la présente invention se rapportent à des unités galéniques résultant de la compression de poudres ou de granules et ont une taille inférieure à 4,5 mm, avantageusement inférieure à 4 mm, plus avantageusement inférieure à 3 mm. Les mini comprimés ont avantageusement une taille supérieure à 0,5 mm, avantageusement supérieure à 1 mm, plus avantageusement supérieure à 2 mm.

Les différents procédés de fabrication de comprimés, par exemple par compression directe ou par compression après granulation par voie humide ou par voie sèche, sont présentés notamment dans *«*Remington's pharmaceutical Sciences, 16 ème Ed, 1980, Mack Publ. Co. of Easton, PA, USA*».*

Les mini comprimés conformes à l'invention peuvent être préparés par compression après granulation.

De façon avantageuse, les mini comprimés selon la présente invention sont obtenus par granulation à sec puis homogénéisation. Le mélange homogénéisé est ensuite soumis à une force de compression qui confère au comprimé résultant une dureté satisfaisante. Plus particulièrement, l'ivermectine micronisée peut être montée sur supports (obtention de microgranules) ou par extrusion-sphéronisation ou granulée par voie humide ou sèche (obtention de granules).

Les granulats d'ivermectine micronisée peuvent être obtenus par granulation par voie sèche ou par voie humide, généralement en présence d'au moins un agent liant et d'un liquide de mouillage le cas échéant, selon des techniques bien connues de l'homme de l'art. Cette étape de granulation améliore l'uniformité de teneur des comprimés fabriqués.

Le mini comprimé peut comprendre un ou plusieurs excipients destinés soit à favoriser le déroulement du processus de compression tels que des agents anti-collage comme la silice colloïdale, le talc, le stéarate de magnésium, le Polyéthylène Glycol (PEG) ou le stéarate de calcium, soit à améliorer la cohésion du comprimé lors de la compression, tels que les agents liants utilisés classiquement dans cette fonction, en particulier les amidons, les dérivés cellulosiques, soit des agents de charge, soit des lubrifiants, soit des plastifiants, soit des agents de remplissage, soit des édulcorants soit des colorants.

De façon avantageuse, chaque mini comprimé selon la présente invention comprend au moins un diluant pharmaceutiquement acceptable. A titre d'exemple de diluant, on peut par exemple citer maltodextrine, poudre de sucre anhydre, amidon, des dérivés de cellulose, notamment de la cellulose microcristalline, de l'éthylcellulose et de l'hydroxypropylméthylcellulose,,des polyols, notamment les polyols de moins de 13 atomes de carbone, en particulier le mannitol, le xylitol, le sorbitol, le maltitol, des gommes, des dérivés de la silice, des dérivés de calcium ou de potassium, des composés minéraux tels que les phosphates dicalciques, des phosphates tricalciques et des carbonates de calcium, du saccharose, la glycine et autres acides aminés pharmaceutiquement compatibles, et leurs dérivés, le lactose et ses dérivés, ou des mélanges de ceux-ci.

De façon avantageuse, chaque mini comprimé selon la présente invention comprend au moins un liant pharmaceutiquement acceptable. A titre d'exemple de liant, on peut par exemple citer de l'amidon, notamment de l'amidon pré-gélatinisé, du saccharose, de la gomme arabique, de la polyvinylpyrrolidone (PVP ou polyvidone), de l'hydroxypropylméthylcellulose (HPMC), de la gomme laque, de l'hydroxypropylcellulose (HPC), de la cellulose, des polyols ou des alginates, des glycérides polyglycolysés (Gelucire^{®} ) ou des macrogolglycérides, notamment macrogolglycérides de stéaroyle, également des dérivés acryliques, ainsi que des mélanges de ceux-ci.

Parmi les polyols, on peut citer notamment le mannitol, le sorbitol, le maltitol ou le xylitol. De façon avantageuse, chaque mini comprimé selon la présente invention comprend au moins un lubrifiant pharmaceutiquement acceptable. A titre d'exemple de lubrifiant, on peut par exemple citer le talc, les dérivés de silice en particulier le gel de silice micronisé, les cires, le stéarate de magnésium, et leurs mélanges, avantageusement le stéarate de magnésium.

De façon avantageuse, chaque mini comprimé comprend un diluant, un liant, un agent antioxydant, un lubrifiant, et de l'ivermectine micronisée.

Avantageusement, chaque mini comprimé comprend, en poids par rapport au poids total du mini comprimé :
- de l'ivermectine micronisée, dans les quantités décrites précédemment ;
- un diluant, en une quantité allant de 70% à 90% en poids, par rapport au poids total du mini comprimé ;
- un liant, en une quantité allant de 5% à 15% en poids, par rapport au poids total du mini comprimé ;
- un agent antioxydant, en une quantité allant de 0,005% à 0,05% en poids, par rapport au poids total du mini comprimé ;
- un lubrifiant, en une quantité allant de 0,5% à 1,5% en poids, par rapport au poids total du mini comprimé.

Avantageusement, on mélange l'ivermectine micronisée avec le ou les excipients de compression, puis on granule ce mélange par voie sèche ou humide afin d'obtenir des granules directement compressibles.

Les mini comprimés peuvent être préparés par un procédé comportant les étapes suivantes :
- mélange de l'ivermectine micronisée avec le ou les excipients,
- éventuellement granulation et
- compression dudit mélange,
- éventuellement enrobage du mini comprimé.

La compression est avantageusement réalisée sur machine à comprimer rotative.

Chaque mini comprimé comprend avantageusement de 0,5 à 2 mg d'ivermectine, avantageusement 1 mg d'ivermectine.

Chaque mini comprimé a avantageusement une dureté variant de 8 à 20 N, avantageusement de 8 à 14 N.

La préparation de mini formulations, avantageusement sous forme de microgranules ou de mini comprimés, qui vont remplir une enveloppe, avantageusement elle-même destinée à être administrée par voie orale telle qu'une gélule ou capsule, de préférence gélule, permet d'adapter facilement la teneur en ivermectine par prise selon la dose recherchée. La préparation de formulation à prise orale unique ayant une concentration plus élevée en ivermectine permet de diminuer le nombre de prises et ainsi d'améliorer l'observance du traitement.

En particulier, la formulation consiste en une formulation de microgranules ou mini comprimés; cette formulation étant ensuite elle-même repartie dans des gélules ou capsules de différentes tailles en fonction du dosage final d'ivermectine souhaité.

Le développement de gélules ou capsules dosées avantageusement de 3 mg à 75 mg, avantageusement de plus de 3 mg à 75 mg, plus avantageusement de plus de 3 mg à 50 mg, plus avantageusement de 12 mg à 50 mg, plus avantageusement de 12 mg à 24 mg, en ivermectine permet d'administrer aux patients une même formulation tout en diminuant le nombre d'unités thérapeutiques absorbées.

L'autre intérêt de la formulation présentée réside dans l'effet pharmacologique supérieur observé comparativement au comprimé oral de référence, le stromectol^{®} ; en effet la formulation présentée permet également une augmentation de la biodisponibilité. La formulation a une biodisponibilité supérieure à 100% comparativement au comprimé de stromectol^{®} pour un même dosage en ivermectine. Ainsi, de manière surprenante la prise d'une formulation pour un dosage donné en ivermectine présente une biodisponibilité supérieure à 100% comparativement à la prise d'une dose identique d'ivermectine sous forme d'un ou plusieurs comprimé(s) de tromectol^{®}. En particulier, l'étude pharmacocinétique effectuée sur les deux formulations microgranules en gélules ou capsules et mini comprimés en gélules ou capsules, comprenant chacune 12 mg d'ivermectine, ont donné respectivement des valeurs de biodisponibilité, notamment une concentration plasmatique maximale (Cmax) de 104 % et 185 % comparativement au comprimé de stromectol^{®} pour un même dosage en ivermectine (soit 4 comprimés de 3 mg d'ivermectine) ce qui correspond à un gain d'activité très supérieure, sans apparition de toxicité.

L'amélioration de la biodisponibilité se voit notamment au travers d'une valeur d'aire sous la courbe de concentration plasmatique en fonction du temps (AUC), du temps zéro (T₀, 0 dans AUC_{0-T}) au moment de la dernière concentration mesurée au-dessus de la limite de quantification, plus élevée lorsqu'une même dose d'ivermectine est administrée sous la forme d'une formulation selon l'invention comparativement à une administration sous la forme d'un ou plusieurs comprimé(s) de stromectol^{®}.

L'amélioration de la biodisponibilité se voit notamment au travers d'un Tₘₐₓ (temps pour atteindre la concentration plasmatique maximale Cₘₐₓ) réduit lorsqu'une même dose d'ivermectine est administrée sous la forme d'une formulation selon l'invention comparativement à une administration sous la forme d'un ou plusieurs comprimé(s) de stromectol^{®}.

L'invention a également pour objet un procédé de préparation d'une formulation galénique selon l'invention, comprenant les étapes suivantes :
a) préparation d'une mini formulation sous forme solide, ayant une taille inférieure à 4,5 mm, d'ivermectine micronisée ;
b) remplissage d'une enveloppe de la formulation avec la quantité désirée de mini formulation solide préparée à l'étape a).

En particulier, l'étape b) est une étape de mise en gélules avec la quantité désirée de mini formulation solide préparée à l'étape a).

L'étape b) permet de faire varier la teneur finale en ivermectine, en faisant varier la quantité de mini formulation, selon le dosage recherché.

La mini formulation et l'enveloppe sont tels que décrits précédemment.

L'invention a en particulier pour objet la préparation d'une mini formulation sous forme solide qui est un ensemble de microgranules préparées à l'étape a) selon le procédé décrit précédemment. En particulier, selon un procédé comprenant les étapes suivantes :
i) préparation d'une suspension comprenant l'ivermectine micronisée, un agent anti-oxydant, un liant et un solvant ;
ii) pulvérisation de la suspension préparée à l'étape ii) sur support solide d'une taille inférieure à 800 µm puis séchage des microgranules non revêtus ainsi obtenus ;
iii) pulvérisation d'une suspension d'enrobage sur les microgranules obtenus suite à l'étape ii) puis séchage des microgranules revêtus ainsi obtenus.

Lors de l'étape ii), la suspension est avantageusement pulvérisée en lit d'air fluidisé à une température comprise en 40°C et 55°C.

Lors de l'étape ii), le support solide est avantageusement pré-chauffé à une température comprise en 40°C et 55°C.

Lors de l'étape iii), la suspension est avantageusement pulvérisée en lit d'air fluidisé à une température comprise en 40°C et 55°C.

Les microgranules, l'enrobage, le support solide, l'agent antioxydant, le liant, le solvant, l'ivermectine micronisé et les appareils mis en jeu sont tels que décrits précédemment.

L'invention a en particulier pour objet la préparation d'une mini formulation sous forme solide qui est un mini comprimé préparé à l'étape a) selon le procédé décrit précédemment, notamment un procédé de granulation humide. En particulier, selon un procédé comprenant les étapes suivantes :
j) mélange d'un agent anti-oxydant et d'une partie du diluant ;
jj) ajout au mélange obtenu suite à l'étape j) de l'ivermectine micronisée, du reste du diluant et du liant puis mélange de l'ensemble ;
jjj) ajout au mélange obtenu suite à l'étape jj) d'une partie du lubrifiant puis mélange de l'ensemble ;
jjjj) granulation à sec du mélange obtenu suite à l'étape jjj) ;
jjjjj) ajout aux particules obtenues suite à l'étape jjjj) du reste du lubrifiant, mélange puis compression pour former les mini comprimés.

Les mini comprimés, le lubrifiant, le diluant, l'agent antioxydant, le liant, le solvant, l'ivermectine micronisé et les appareils mis en jeu sont tels que décrits précédemment. L'invention a également pour objet l'utilisation d'une formulation galénique telle que décrite pour adapter de manière homothétique le dosage en ivermectine en fonction de la posologie.

L'invention permet d'offrir au patient une formulation galénique adaptée à son poids. Ainsi, le traitement pourra s'effectuer par administration par voie orale d'une seule formulation galénique par prise; au lieu de nécessiter d'avaler plusieurs comprimés à chaque prise. Ce traitement permet un ajustement plus fin de la posologie par rapport au poids du patient. Ce traitement permet ainsi également une amélioration de l'observance.

L'invention a également pour objet une formulation galénique telle que décrite pour son utilisation dans le traitement ou la prévention de maladies dues ou induites par un parasite, une bactérie, un virus.

En particulier, la formulation peut être utilisée dans le traitement ou la prévention des parasitoses, notamment de l'onchocercose, des filarioses, des pédiculoses, ou de la gale. En particulier, la formulation peut être utilisée dans le traitement ou la prévention d'infections bactériennes, telles que les maladies nosocomiales dont certaines infections au staphylocoque doré *(staphylococcus aureus*)*.*

En particulier, la formulation peut être utilisée dans le traitement ou la prévention d'infections virales telles que la grippe ou les maladies à coronavirus notamment la COVID-19. En particulier, les virus peuvent être des SRAS-CoV ou des MERS-CoV.

L'invention a également pour objet une formulation galénique telle que décrite pour son utilisation dans la préparation d'un médicament destiné au traitement ou à la prévention de maladies dues ou induites par un parasite, une bactérie, un virus.

En particulier, le médicament est destiné au traitement ou à la prévention des parasitoses, notamment de l'onchocercose, des filarioses, des pédiculoses, ou de la gale.

En particulier, le médicament est destiné au traitement ou à la prévention d'infections bactériennes, telles que les maladies nosocomiales et les infections au staphylocoque doré *(staphylococcus aureus*)*.*

En particulier, le médicament est destiné au traitement ou à la prévention d'infections virales telles que la grippe ou les maladies à coronavirus notamment la COVID-19. En particulier, les virus peuvent être des SRAS-CoV ou des MERS-CoV.

L'invention a également pour objet une méthode de traitement thérapeutique, ou de prévention, de maladies dues ou induites par un parasite, une bactérie, un virus comprenant l'administration, à une personne en ayant besoin, d'une formulation galénique telle que décrite précédemment. Avantageusement, on administre une formulation par prise. En effet, l'invention permet un ajustement plus fin de la posologie par rapport au poids du patient, ce qui est par ailleurs favorable à l'observance du traitement.

En particulier, la maladie peut être une parasitose, notamment de l'onchocercose, des filarioses, des pédiculoses, ou de la gale.

En particulier, la maladie peut être une infection bactérienne, telle que les maladies nosocomiales et les infections au staphylocoque doré *(staphylococcus aureus*)*.*

En particulier, la maladie peut être une infection virale telle que la grippe ou les maladies à coronavirus notamment la COVID-19. En particulier, les virus peuvent être des SRAS-CoV ou des MERS-CoV.

Dans tous ces traitements curatifs ou préventifs, le patient est avantageusement l'homme. La formulation ici décrite permet en particulier d'améliorer l'observance du traitement. En effet, par un ajustement plus fin du dosage dans la formulation en fonction du poids du patient, une seule formulation par prise doit être avalée par le patient. Pour le traitement d'infections parasitaires, la dose recommandée est avantageusement de 200 µg d'ivermectine par kg en une prise unique. Dans le cas de certains vers tropicaux, la prise unique peut être renouvelée 6 mois après. Dans le cas de la gale, il peut être nécessaire de renouveler le traitement après 8 à 15 jours.

Pour le traitement d'infections bactériennes ou virales, la dose recommandée en ivermectine est avantageusement de 24 mg à 70 mg minimum par jour, avantageusement sur 5 à 15 jours de traitement.

### Exemples

Deux types gélules selon l'invention sont préparées.

Dans un premier type (A), les gélules comprennent des microgranules et ont la composition suivante :

**Tableau 1**

| Ingrédient | Composition (%) | Composition par unité (mg) |
|---|---|---|
| Ivermectine micronisée | 10,89 | 12,000 |
| Billes de sucre (500-600 µm) | 82,52 | 90,912 |
| Hypromellose E5 | 2,72 | 3,001 |
| BHA | 0,019 | 0,021 |
| Opadry AMB II white | 3,85 | 4,237 |
| Capsules gélatine dures taille #4 | n.a. | 38,00 |
| total | 100 | 148,171 |

n.a. non applicable

Ces microgranules sont préparés selon le procédé suivant :
a) préparation d'une dispersion d'ivermectine selon les étapes successives suivantes :
   - dissolution de l'hypromellose E5 dans de l'eau purifiée jusqu'à obtention d'une solution limpide ;
   - ajout de l'ivermectine micronisée à la solution précédemment obtenue ;
   - tamisage de la suspension obtenue précédemment au travers d'un filtre à 0,35 mm ;
b) préparation d'une solution de BHA par ajout de BHA dans de l'éthanol à 96% en volume jusqu'à obtention d'une solution limpide ;
c) ajout de la solution de BHA à la dispersion d'ivermectine obtenue en a) sous agitation ;
d) pulvérisation en lit d'air fluidisé (température de l'air injecté : 53°C) de la suspension obtenue en c) sur le support neutre (billes de sucres pré-chauffées à 39°C) ;
e) séchage des particules obtenues en d) (température de l'air injecté : 50°C) puis tamisage sur grille à 0,8 mm ;
f) préparation d'une suspension d'Opadry AMB II suivant les indications du fournisseur ;
g) enrobage des particules obtenues en e) par la suspension obtenue en f) en lit d'air fluidisé (température de l'air injecté : 53°C) ;
h) séchage des particules obtenues en g) (température de l'air injecté : 50°C) puis tamisage sur grille à 0,8 mm ;
i) mise en gélules avec les microgranules obtenus en h).

Dans un deuxième type (B), les gélules comprennent des mini comprimés et ont la composition suivante :

**Tableau 2**

| Ingrédient | Composition (%) | Composition par unité (mg) |
|---|---|---|
| Ivermectine micronisée | 12,50 | 12,000 |
| Cellulose microcristalline | 76,48 | 73,421 |
| Amidon maïs pré-gélatinisé | 10,00 | 9,600 |
| BHA | 0,02 | 0,019 |
| Stéarate de magnésium | 1,00 | 0,960 |
| Capsules gélatine dures taille #4 | n.a. | 38,00 |
| total | 100 | 134,000 |

Ces mini comprimés sont préparés selon le procédé suivant :
a) préparation d'une solution de BHA par ajout de BHA dans de l'éthanol à 96% en volume jusqu'à obtention d'une solution limpide ;
b) mélange dans un granulateur de la solution de BHA obtenue en a) à une partie de la cellulose microcristalline (55% en poids par rapport au poids total de la cellulose microcristalline) ;
c) ajout et tamisage à 1 mm de : la moitié en poids du mélange obtenu en b), puis l'ivermectine micronisée, puis la deuxième moitié en poids du mélange obtenu en b), puis la deuxième partie de la cellulose microcristalline (45% en poids par rapport au poids total de la cellulose microcristalline), puis l'amidon prégélatinisé ; puis mélange de l'ensemble ;
d) ajout d'une partie du stéarate de magnésium au mélange obtenu en c) puis mélange et granulation à sec ;
e) ajout de l'autre partie du stéarate de magnésium au mélange obtenu en d) puis mélange ;
f) compression du mélange obtenu en e)
g) mise en gélules avec les mini comprimés obtenus en f).

La pharmacocinétique des gélules type A (gélule remplie de microgranules) et de gélules type B (gélule remplie de mini comprimés) est évaluée et comparée au produit de référence Stromectol^{®} après une administration orale unique à jeun chez l'homme. Chaque comprimé de Stromectol^{®} comprend 3mg d'ivermectine.

13 patients (sujets humains mâles et femelles adultes en bonne santé) sont inclus par étude de pharmacocinétique.

Une dose orale unique de 12 mg d'ivermectine a été administrée à jeun au cours de chaque période d'étude. Dans chaque période d'étude, 22 échantillons de sang ont été prélevés pour chaque sujet. Le premier échantillon de sang a été prélevé avant l'administration du médicament tandis que les autres ont été prélevés jusqu'à 36 heures après l'administration du médicament. Les sujets ont été confinés au site clinique au moins 10 heures avant chaque administration jusqu'à au moins 24 heures après chaque administration. Les sujets reviennent au site clinique pour l'échantillon de sang restant.

### Critères d'évaluation :

### Pharmacocinétique (PK):

Les concentrations plasmatiques d'ivermectine B1a ont été mesurées pour déterminer les paramètres pharmacocinétiques suivants :
- La concentration plasmatique maximale (Cₘₐₓ) ;
- L'aire sous la courbe de concentration plasmatique en fonction du temps (AUC) du temps zéro (T₀, 0 dans AUC_{0-T}) au moment de la dernière concentration mesurée au-dessus de la limite de quantification (noté T_{LQC}, T dans AUC_{0-T}) (cette aire sous la courbe de T₀ à T_{LQC} est notée AUC_{0-T})

Sécurité : Les évaluations de sécurité comprenaient un examen physique, des signes vitaux, des tests de laboratoire clinique et une surveillance des événements indésirables.

### Méthodes statistiques :

Une analyse non compartimentale (NCA) avec une hypothèse de phase terminale log-linéaire a été utilisée pour l'analyse PK. La règle trapézoïdale a été utilisée pour estimer l'AUC.
- Les concentrations plasmatiques d'ivermectine B1a ont été moyennées par instant et par traitement en utilisant la moyenne arithmétique, la moyenne géométrique, le minimum, la médiane, le maximum, l'écart type et le coefficient de variation (CV) ;
- Cₘₐₓ, Tₘₐₓ (temps pour atteindre la concentration plasmatique maximale Cₘₐₓ) et AUC_{0-T} et T_{LQC} ont été déterminés par patient et ont été moyennés par traitement en utilisant la moyenne arithmétique, la moyenne géométrique, le minimum, la médiane, le maximum, l'écart type et le CV.

L'analyse statistique a été réalisée en utilisant une analyse de variance à effets mixtes (ANOVA) des paramètres PK ; l'intervalle de confiance (IC) bilatéral à 90% du rapport des moyennes géométriques pour la Cₘₐₓ et l'AUC_{0-T} était basée sur des données transformées par In.

Tₘₐₓ a été analysé en utilisant une approche non paramétrique.

### Modèle ANOVA:

Facteurs fixes : séquence, période, traitement et sujet (imbriqués dans la séquence)

Une étude croisée à un seul centre, randomisée, à dose unique, en aveugle en laboratoire, sur 3 périodes, 3 séquences, à jeun avec une période de sevrage de 28 jours entre les traitements a été menée chez 13 sujets sains de sexe masculin et féminin.

Les traitements suivants ont été administrés par voie orale :
- traitement INV1 : une dose de gélule type A (12 mg d'ivermectine)
- traitement INV2 : une dose de gélule type B (12 mg d'ivermectine)
- traitement REF : 4 comprimés Stromectol^{®} (4x3 = 12 mg d'ivermectine)

Le taux et le degré d'absorption de l'ivermectine B1a ont été mesurés et comparés après une dose unique (1 capsule de 12 mg) du Test (Traitement INV1 et Traitement INV2) et (4 comprimés de 3 mg) des formulations de référence (Traitement REF). Pour évaluer la pharmacocinétique de l'étude, des échantillons de sang ont été prélevés 36 heures après l'administration.

Les résultats sont présentés dans les tableaux suivants :

**Tableau 3**

| Paramètres (Unités) | traitement INV1 | traitement INV2 | traitement REF |
|---|---|---|---|
| | Moyenne (CV%) | Moyenne (CV%) | Moyenne (CV.%) |
| Cₘₐₓ (ng/mL) | 57,715 (31,4) | 34,32 (43,4) | 35,43 (51,8) |
| Tₘₐₓ (heures) | 2,67 (2,00 - 5,00) | 3,67 (2,33 - 6,00) | 4,67 (3,00 - 5,50) |
| AUC_{0-T} (ng.h/mL) | 598,779 (30,5) | 417,447 (40,5%) | 399,394 (48,3) |

**Tableau 4**

| Paramètres (Unités | CV intra sujets (%) | Moyenne géométrique par les moindres carrés | | Ratio | Intervalle de confiance à 90% | |
|---|---|---|---|---|---|---|
| | | traitement INV1 | traitement REF | (%) | Inférieur | supérieur |
| Cₘₐₓ (ng/mL) | 34,4 | 54,162 | 29,264 | 185,08 | 145,69 | 235,11 |
| AUC_{0-T} (ng.h/mL) | 35,2 | 571,610 | 334,974 | 170,64 | 133,62 | 217,93 |

**Tableau 5**

| Paramètres (Unités | CV intra sujets (%) | Moyenne géométrique par les moindres carrés | | Ratio | Intervalle de confiance à 90% | |
|---|---|---|---|---|---|---|
| | | traitement INV2 | traitement REF | (%) | Inférieur | supérieur |
| Cₘₐₓ (ng/mL) | 27,8 | 30,605 | 29,264 | 104,58 | 86,07 | 127,08 |
| AUC_{0-T} (ng.h/mL) | 22,2 | 377,615 | 334,974 | 112,73 | 96,35 | 131,89 |

### Comparaison Traitement INV1 vs Traitement REF

Le temps pour atteindre la concentration plasmatique maximale (Tₘₐₓ) est de 2,67 heures pour le traitement INV1 alors qu'il est de 2 heures supplémentaires pour traitement-REF à 4,67 heures. Sur la base de la moyenne géométrique par les moindres carrés, la concentration plasmatique maximale d'ivermectine B1a (Cₘₐₓ) pour le traitement INV1 est environ 85% plus élevée que la Cₘₐₓ après l'administration du traitement REF. L'exposition moyenne globale à l'ivermectine B1a du Traitement INV1 est 70% plus élevée que l'exposition globale du Traitement REF.

### Comparaison Traitement INV2 vs Traitement REF

Le temps pour atteindre la concentration plasmatique maximale (Tₘₐₓ) est de 3,67 heures pour le traitement INV2 alors qu'il faut 1 heure de plus après l'administration du traitement REF à 4,67 heures.

### Effets indésirables :

Aucun événement indésirable grave ni décès n'a été signalé pour aucun des sujets traités dans cette étude. Les quelques effets indésirables rencontrés au cours de l'étude ont été considérés comme non liés au traitement. L'effet indésirable le plus fréquemment ressenti était le mal de tête rapporté par 1 sujet du groupe traitement INV1 et du groupe traitement REF. Tous les autres effets indésirables ont été vécus par pas plus d'un sujet dans n'importe quel groupe de traitement.

Tous les effets indésirables rencontrés au cours de l'étude ont été jugés d'intensité légère. Aucun des sujets n'a présenté d'effets indésirables modérés ou sévères au cours de l'étude. Aucune anomalie cliniquement significative des paramètres de laboratoire, des signes vitaux et des examens physiques n'a été notée au cours de l'étude.

Dans l'ensemble, les médicaments testés étaient généralement sûrs et bien tolérés par les sujets inclus dans cette étude.

## Revendications

1. Formulation galénique, adaptée à une administration par voie orale, comprenant une enveloppe renfermant des mini formulations sous forme solide, ayant une taille inférieure à 4,5 mm, d'ivermectine micronisée.

2. Formulation galénique selon la revendication 1, dans laquelle l'ivermectine micronisée a une distribution de tailles de particules dans laquelle le D50 varie entre 1 µm et 30 µm, préférentiellement entre 1 µm et 20 µm.

3. Formulation galénique selon la revendication 1 ou 2, dans laquelle la formulation galénique comprenant une enveloppe est choisie parmi des gélules, des sachets ou des sticks.

4. Formulation galénique selon l'une quelconque des revendications précédentes, comprenant 3 mg à 75 mg d'ivermectine par formulation, avantageusement de plus de 3mg à 75 mg d'ivermectine par formulation.

5. Formulation galénique selon l'une quelconque des revendications précédentes, dans laquelle la mini formulation solide comprend de 2% à 50% en poids, par rapport au poids total de la mini formulation, d'ivermectine micronisée.

6. Formulation galénique selon l'une quelconque des revendications précédentes, dans laquelle les mini formulations solides sont choisies parmi des microgranules ou des mini comprimés.

7. Formulation galénique selon la revendication 6, dans laquelle chaque microgranule comprend du centre vers la périphérie
- un support,
- au moins une couche de montage comprenant l'ivermectine micronisée, optionnellement un agent antioxydant, et un agent liant pharmaceutiquement acceptable,
- au moins une couche d'enrobage.

8. Formulation galénique selon la revendication 6, dans laquelle chaque mini comprimé comprend un diluant, un liant, un agent antioxydant, un lubrifiant, et de l'ivermectine micronisée.

9. Formulation galénique selon la revendication 6 ou 8, dans laquelle chaque mini comprimé a une taille inférieure à 3 mm.

10. Procédé de préparation d'une formulation galénique selon l'une quelconque des revendications précédentes, comprenant les étapes suivantes :
a) préparation d'une mini formulation sous forme solide, ayant une taille inférieure à 4,5 mm, d'ivermectine micronisée ;
b) remplissage d'une enveloppe de la formulation avec la quantité désirée de mini formulation solide préparée à l'étape a).

11. Utilisation d'une formulation galénique selon l'une quelconque des revendications 1 à 9 pour adapter de manière homothétique le dosage en ivermectine en fonction de la posologie.

12. Formulation galénique selon l'une quelconque des revendications 1 à 9 pour son utilisation dans le traitement ou la prévention de maladies dues ou induites par un parasite, une bactérie, un virus.

13. Formulation selon la revendication 12 pour son utilisation dans le traitement ou la prévention des parasitoses, notamment de l'onchocercose, des filarioses, des pédiculoses, ou de la gale.

14. Formulation selon la revendication 12 pour son utilisation dans le traitement ou la prévention d'infections bactériennes, telles que les maladies nosocomiales, notamment certaines infections au staphylocoque doré *(staphylococcus aureus).*

15. Formulation selon la revendication 12 pour son utilisation dans le traitement ou la prévention d'infections virales telles que la grippe ou les maladies à coronavirus notamment la COVID-19.
